# EUROPEAN PATENT APPLICATION

(11) **EP 1 707 576 A1**
(43) Date of publication of application: **04.10.2006**
(21) Application number: 04819907.9
(22) Date of filing: 02.12.2004
(51) Int. Cl.: C07K 16/44, C12N 5/18, C12P 21/08, G01N 33/53

(54) **ANTIBODIES RECOGNIZING METHYLLYSINE, PROCESS FOR PRODUCING THE SAME AND UTILIZATION THEREOF**

(30) Priority: 02.12.2003 JP 2003403313
(71) Applicant: Advanced Life Science Institute, Inc., Wako-shi, Saitama, 351-0112 (JP)
(72) Inventor: KOMATSU, Yasuhiko, Ageo-shi, Saitama 362-0042 (JP); IWABATA, Hisako, Saitama-shi, Saitama 330-0061 (JP)
(74) Representative: TER MEER - STEINMEISTER & PARTNER GbR
(86) International application number: PCT/JP2004/017959
(87) International publication number: WO 2005/054296

(57) **Abstract**

Antibodies capable of recognizing many types of proteins having methyllysine residues are established by immunizing an animal with a chemically methylated protein other than histone and subsequent screening or the like depending on the reactivity to a protein obtained by chemically methylating a protein other than the protein used in the immunization. A process for producing such an antibody is also established. These antibodies are useful in searching for and studying various methylated proteins and are particularly useful in regulating the functions of biological molecules wherein the methylation of a lysine residue plays an important role, and in diagnosing a disease by detecting a methyllysine-containing protein.

## Description

### Technical Field

The present invention relates to an antibody which can be used in detection of methyllysine as one of post-translational protein modifications, a process for producing the same, and a method of detecting a methylated protein by using the antibody.

### Background Art

It is known that proteins after translation do not exhibit their functions in a translated form and undergo various post-translational modifications. For example, the phosphorylation of proteins is important as a signal cascade for transmission of extracellular signals to nuclei or as a regulatory factor for progress of normal cell cycle, and the acetylation of histone is important for efficient progress of transcription. Proteins when conjugated with ubiquitin are transferred to proteasome and degraded to loose their activity. On one hand, signal peptides are cut off with signal peptidases present in endoplasmic reticulum membrane to make many proteins active. In this way, proteins undergo various post-translational modifications thereby exhibiting their respective functions at a suitable time in a suitable place.

Among post-translational protein modifications coming to attract attention in recent years, there is the methylation of lysine residues in proteins. It is known that in a core histone molecule, for example, lysine residues in H3 and H4 are methylated, and it is reported that while the methylation of lysine at the fourth position in H3 is positively correlated with acetylation of H3 and an activated site of a gene, the methylation of lysine at the ninth position in H3 is positively correlated with low acetylation of H3 and a suppressed site of gene expression (M. D. Litt, et al., Science (US), 2001, vol. 293, p. 2453). It is also reported that a heparin-binding hemagglutinin (HBHA) molecule important for adhesion of tubercle bacillus to an epithelial cell, upon methylation of its heparin-binding region, increases resistance to protease (K. Pethe, et al., Proc. Natl. Acad. Sci. USA, 2002, vol. 99, p. 10759). Further, it is known that in elongation factor 1α (EF-1α) catalyzing GTP-dependent ribosome binding of aminoacyl-tRNA molecule, some lysine residues in the molecule thereof are methylated (see, for example, T. E. Dever, et al., J. Biol. Chem. (US), 1989, vol. 264, p. 20518), and it is also reported that by this methylation, EF-1α activity is enhanced (W. A. Fonzi, et al., Mol. Cell. Biol. (US), 1985, vol. 5, p. 1100). However, methyllysine-containing proteins found so far are not so many, and there is no great advance on study of a question how important role the functional regulation of proteins by methylation plays in the living body and what relationship with diseases the functional regulation has. Findings of various methyllysine-containing proteins and elucidation of functions thereof in the future are expected under the present circumstance.

As effective means for detecting post-translational protein modification, use of a modification-specific probe molecule is anticipated. For example, various antibodies recognizing phosphorylated tyrosine are commercially available and widely utilized. Antibodies useful in the future in finding new modified proteins are considered to be antibodies that can, without so much depending on surrounding amino acid sequences, recognize only objective modified amino acids to the maximum degree. Antibodies having such properties are developed and commercially available for phosphorylated tyrosine. However, such antibodies are not reported for methyllysine. The present situation of anti-methyllysine antibodies reported so far is as follows:

With respect to histone, its methylated site-specific function attracts attention as described above, and for the purpose of analyzing the methylation site-specifically, anti-methylated histone antibodies (International Publication No. 02/18418) are developed and commercially available (available from Santa Cruz, etc.), but as a matter of course, these antibodies are not those recognizing various kinds of methyllysine-containing proteins. An anti-methyllysine antibody (rabbit polyclonal antibody) obtained by using a chemically methylated histone H1 molecule as immunogen is also developed and commercially available (abcamLtd.), but this antibody can recognize only dimethyllysine and is poor in an ability to recognize methylated proteins other than histone. It is also reported that a monoclonal antibody mAb 4057D2 having an ability to prevent tubercle bacillus from spreading from the lung by recognizing an HBHA molecule in the tubercle bacillus recognizes a methylated HBHA molecule, but does not recognize a non-methylated HBHA molecule and can also recognize a methylated version of a laminin-binding protein which similar to HBHA, has a lysine-rich domain. Accordingly, this antibody is considered to recognize a methyllysine cluster present in the protein. However, the ability of this antibody to recognize othermethylated proteins not having a lysine-rich domain is considered not to be so strong (K. Pethe, et al., Proc. Natl. Acad. Sci. USA, 2002, vol. 99, p. 10759). As described above, there is no report of teaching or suggesting antibodies which can, without being influenced by surrounding amino acids, recognize a methyllysine residue in a protein, and there is no established method in which various methylated proteins can be recognized.

As described above, finding of various methyllysine-containing proteins and analysis of functions thereof are expected, and under this circumstance, there is strong need for a method which can, without so depending on the type of its surrounding amino acids, recognize methyllysine residues estimated to occur in various proteins. If such method could be established, it is expected that the study on methyllysine-containing proteins can be rapidly developed.

### Disclosure of Invention

The object of this study is to acquire a method which can recognize methyllysine residues in a protein, without being so influenced by the type of the protein or the type of surrounding amino acids. Particularly, a main object of the present invention is to acquire an anti-methyllysine antibody which can be used as a probe molecule for the method. A further object of the present invention is to provide means of obtaining an antibody having such properties.

The present inventors made a wholehearted endeavor based on the hypothesis that antibodies induced to be produced by immunizing an animal with a chemically methylated protein as an immunogen contain an antibody which can, without being influenced by surrounding amino acid residues, can recognize a methyllysine residue, and as a result, they succeeded in purifying an antibody having such properties or in producing the antibody as a monoclonal antibody.

That is, the problem described above can be solved by the following means:
(1) An anti-methyllysine antibody capable of specifically recognizing methyllysine and not recognizing lysine.
(2) An anti-methyllysine antibody capable of specifically recognizing a methyllysine residue in a protein, without being influenced by surrounding amino acid residues.
(3) An anti-methyllysine antibody binding specifically to dimethyllysine and monomethyllysine.
(4) An anti-methyllysine antibody obtained by a method comprising a step of immunizing an animal by using, as an antigen, a protein prepared by chemically methylating a protein other than histone.
(5) The antibody according to the above-mentioned 4, wherein the protein other than histone is selected from keyhole limpet hemocyanin, bovine serum albumin, and ovalbumin.
(6) The antibody according to any of the above-mentioned 1 to 5, whose reactivity to dimethyllysine is superior to reactivity to monomethyllysine.
(7) The antibody according to any of the above-mentioned 1 to 6, which is a polyclonal antibody.
(8) The antibody according to any of the above-mentioned 1 to 6, which is a monoclonal antibody.
(9) The antibody according to any of the above-mentioned 1 to 6, which is a rabbit polyclonal antibody.
(10) The antibody according to any of the above-mentioned 1 to 6, which is a mouse monoclonal antibody.
(11) A hybridoma producing an anti-methyllysine antibody and selected from the group consisting of MEK3D7, MEK4E10, MEK5F7, MEK2-5A11 and MEK2-5B11.
(12) An anti-methyllysine mouse monoclonal antibody produced by the hybridoma according to the above-mentioned 11.
(13) A process for producing the antibody according to the above-mentioned 1 to 10 or 12, which comprises immunizing an animal by using, an antigen, a protein obtained by chemically methylating a protein other than histone.
(14) The process according to the above-mentioned 13, wherein the protein is selected from keyhole limpet hemocyanin, bovine serum albumin, and ovalbumin.
(15) The process according to the above-mentioned 13 or 14, which comprises a step of checking an antibody titer by examining reactivity to a protein obtained by chemically methylating a protein different from the protein used in immunization.
(16) The process according to the above-mentioned 15, wherein the protein used in immunization is keyhole limpet hemocyanin and the protein used for checking an antibody titer is bovine serum albumin.
(17) The process according to any of the above-mentioned 13 to 16, wherein an affinity column having a chemically methylated protein immobilized thereon is used in the step of purification.
(18) The process according to the above-mentioned 13 to 17, wherein the animal is a rabbit.
(19) The process according to the above-mentioned 13 to 17, wherein the animal is a mouse.
(20) A process for producing the polyclonal antibody according to the above-mentioned 7 or 9, which comprises immunizing an animal with an antigen obtained by chemically methylating a protein and subjecting the resulting antibody to affinity purification with a protein obtained by chemically methylating a protein different from the antigen.
(21) A process for producing the monoclonal antibody according to the above-mentioned 8 or 10, which comprises immunizing an animal with an antigen obtained by chemically methylating a protein and then selecting a hybridoma secreting an antibody recognizing a protein obtained by chemically methylating a protein different from the antigen.
(22) A method of detecting a methylated protein, which comprises using the antibody according to the above-mentioned 1 to 10 or 12.

The anti-methyllysine monoclonal antibody and polyclonal antibody provided according to the present invention are useful in searching for a new methyllysine-containing protein which can serve as a cause of disease or as a diagnostic marker. Further, the anti-methyllysine monoclonal antibody and polyclonal antibody provided according to the present invention are also useful in regulating the functions of a biological molecule wherein the methylation of a lysine residue plays an important role, or in diagnosing a disease by detecting a methyllysine-containing protein. The process for producing an anti-methyllysine antibody provided according to the present invention is useful in obtaining an anti-methyllysine antibody having an activity stronger than conventional ones and having a broader allowable range of amino acid sequences surrounding a methyllysine residue.

### Antibody capable of recognizing methyllysine in a protein, without being influenced by surrounding amino acids

### (1) Monoclonal antibody

An animal is immunized with a chemically methylated protein as an immunogen, and then a hybridoma producing a monoclonal antibody is produced in a usual manner. A hybridoma producing a monoclonal antibody recognizing a methyllysine residue is screened by measuring reactivity to a protein obtained by methylating a protein different from the protein used as immunogen. By screening with a methylated protein different from the methylated protein used as immunogen, antibodies recognizing methyllysine residues in various proteins not limited to a specific protein can be obtained.

The method of chemically methylating a protein includes, but is not limited to, a method of allowing formaldehyde to act in the presence of sodium borohydride under the condition of pH 7 or more.

The protein used as immunogen includes, but is not limited to, experimentally generally used proteins such as keyhole limpet hemocyanin, bovine serum albumin, ovalbumin, human serum albumin, transferrin, and thyroglobulin. Unlike the histone protein, these proteins are desirably those free of a lysine-rich domain.

The histone protein has a large amount of lysine residues as a cause of its strong basicity, and many lysine-rich domains occur in every histone protein. Accordingly, it is considered that when chemically methylated histone is used as immunogen, antibodies to methyllysine-rich domains are predominantly produced. Actually, a rabbit anti-methyllysine antibody (Abcam Ltd.) obtained by using an antigen prepared by chemically methylating histone H1 is excellent in reactivity to histone, but is not always excellent in reactivity to other methylated proteins. Accordingly, a protein having strong basicity with many lysine-rich domains should not be used as the protein methylated for use as immunogen .

As the protein used for checking an antibody titer, a protein different from the protein used in immunization can be similarly chemically methylated and used. For example, when methylated KLH is used as antigen, the titer value can be checked by using any of proteins obtained by chemically methylating proteins which include, but are not limited to, bovine serum albumin, ovoalbumin, human serum albumin, transferrin, and thyroglobulin. For checking the antibody titer, a protein obtained by binding methyllysine by various methods to a protein other than the protein used in immunization, an ELISA plate having methyllysine bound directly thereon, or a methyllysine-containing synthetic peptide, natural peptide or natural protein can be used.

### (2) Polyclonal antibody

An animal is immunized with a chemically methylated protein as immunogen to give an antiserum. The resulting antiserum is purified by an affinity column having a methylated protein immobilized thereon, whereby an antibody specifically recognizing the methylated protein can be obtained. By using the affinity column on which a methylated protein different from the protein used as immunogen is immobilized, antibodies capable of broadly recognizing methylated proteins other than the immunogen protein can be obtained.

For example, serum derived from an animal immunized with methylated KLH is purified by an affinity column having methylated BSA immobilized thereon, whereby antibodies specifically recognizing a methylated site can be obtained. As the affinity column, a column having methyllysine bound directly to gel or a column containing a methyllysine-containing synthetic peptide, natural peptide or natural protein bound to gel can also be used.

Further, antibodies binding to non-methylated materials are removed by an affinity column on which a non-methylated version of the protein used as immunogen is immobilized, whereby the specificity for the methylated protein can be improved.

### Method of detecting methylated protein

By immune measurement methods or immune detection methods using the antibody of the present invention, a wide variety of methylated proteins can be measured and detected. These methods include enzyme immunoassay (EIA), radioimmunoassay (RIA), fluoroimmunoassay (FIA), and a latex aggregation method with polystyrene particles sensitized with the antibody. The present antibody can also be used in Western blotting or in staining of various tissues.

### Effect of Invention

Since the antibodies of the present invention can, without being influenced by surrounding amino acid residues, recognize methyllysine residues, these antibodies can be used to provide a method capable of detecting larger kinds of methylated proteins than by conventional methods. Further, these antibodies can be used to regulate functions of biological molecules wherein the methylation of lysine residues plays an important role or to diagnose diseases by detection of methyllysine-containing proteins.

### Best Mode for Carrying Out the Invention

Hereinafter, the preparation of an anti-methyllysine antibody by using keyhole limpet hemocyanin (KLH) as a protein chemically methylated for use as antigen is described in more detail, but is naturally not intended to limit the mode for carrying out the invention.

Chemically methylated KLH can be prepared for example by the following method. 10 mg KLH is dissolved in 1 ml borate buffer (20 mM Na₂B₄O₇, pH 9.3), and 0.5 mg sodium borohydride is added thereto. 0.5 µl formalin is added 5 times at 5-minute intervals, and the mixture is further reacted at room temperature for 30 minutes. After the reaction is finished, the solvent in the reaction solution is exchanged with PBS in a gel filtration column.

A method of immunizing an animal is described on rabbit and mouse as examples. The methylated KLH solution and an adjuvant such as Freund complete adjuvant or TiterMax Gold (CytRx Ltd.) are mixed at a ratio of 1 : 1 and then formed into an emulsion by sonication or by passing the mixture repeatedly through an interchange joint connecting two injection tubes with each other. The prepared antigen-containing emulsion is injected subcutaneously, intradermally, intramuscularly or intraperitoneally at one site or a plurality of sites. One to four weeks after the first immunization, second immunization is carried out in the same manner. When Freund complete adjuvant is used in the first immunization, Freund incomplete adjuvant is desirably used in second and subsequent immunization. The immunization is continued in the same manner until the antibody titer of the anti-methyllysine antibody in blood increases.

Measurement of the antibody titer can be carried out in the following manner. Bovine serum albumin methylated in the same manner as for KLH is dissolved at a concentration of 10 µg/ml in PBS, then added to each well of a 96-well ELISA plate in a volume of 50 µl/well, and adsorbed onto each well overnight at 4°C. Each well is washed with PBS containing 0.05% Tween 20 (PBST) and then used in assay. Before assay, each well may be blocked with PBST containing 1% BSA or the like. In the case of mouse, blood is collected through an orbit venous plexus, a tail vein or a tail artery, or in the case of rabbit, blood is collected through a pinna vein or an ear artery, and the resulting blood is diluted 30-fold with PBST and then separated by centrifugation. The resulting supernatant is used to prepare serial dilutions with PBST, and each dilution is added to each well of the methylated BSA-coated ELISA plate in a volume of 50µl/well. After incubation at room temperature for 30 minutes, each well is washed with PBST, and a horseradish peroxidase(HRP)-labeled anti-mouseIgGsolution diluted suitably with PBST is added to each well in a volume of 50 µl/well. After further incubation at room temperature for 30 minutes, a solution of HRP substrate such as o-phenylenediamine is added for coloration.

When the mouse monoclonal antibody is to be prepared, a sufficient increase in titer is confirmed, then the spleen is removed, and spleen cells are isolated. The cells are fused with separately cultured mouse myeloma (for example, SP2/0-Ag14 etc.) by using polyethylene glycol etc. The successfully fused cells are cultured and selected in HAT (hypoxanthine/aminopterin/thymidine) medium. While the cells are cultured for about 7 to 14 days, half of the medium is exchanged with a fresh medium every several days, and then the antibody titer of the culture supernatant is measured. The cells in the positive wells are cloned by the limiting dilution method to give the objective antibody-producing hybridoma. The hybridoma clones obtained by the method described above can include those exemplified by MEK3D7 (Accession No. FERM P-19595), MEK4E10 (Accession No. FERM P-19596), MEK5F7 (AccessionNo. FERM P-19597), MEK2-5A11 (Accession No. FERM P-19593) and MEK2-5B11 (Accession No. FERM P-19594).

For obtaining the rabbit polyclonal antibody, a sufficient increase in titer is confirmed, then blood is collected, and an anti-methyllysine antibody-containing serum is prepared.

The purified antibody can be obtained by the following procedure. In the case of the monoclonal antibody, a culture solution wherein the hybridoma was cultured can be used as a source subjected to antibody purification. For obtaining a larger amount of the antibody, the hybridoma is transplanted intraperitoneally in a mouse to produce ascitic fluid, whereby a sample containing the anti-methyllysine antibody at high concentration can be obtained in the ascitic fluid, so the ascitic fluid can be used as a starting material to be purified. In the case of the polyclonal antibody, serum containing the antibody is used.

The antibody can be purified with an affinity column on which a methylated protein different from the antigen used in immunization has been immobilized. The affinity column having the methylated protein immobilized thereon can be obtained for example by the following method. A protein such as bovine serum albumin, different from the protein used in immunization, is immobilized on a carrier such as agarose or Sepharose activated with aldehyde or N-hydroxysuccinimide. Then, the immobilized protein is methylated by using formalin and sodium borohydride. By this procedure, an affinity column having the methylated protein immobilized thereon can be obtained. For preparing the affinity column, methyllysine itself may be immobilized on gel, or a methyllysine-containing peptide may be immobilized. A solution containing the antibody is passed through the affinity column prepared by the above method, and the column is washed sufficiently with a buffer such as PBS and then subjected to elution under acid conditions to give the purified antibody.

Before or after the affinity purification, the antibody may be purified with a column having an antibody-binding protein such as protein G, protein A or protein L immobilized thereon.

The fact that the antibody obtained by the above method recognizes methyllysine residues, without significantly depending on surrounding amino acid sequences, can be confirmed for example by the following method. First, the recognition of methyllysine residues by the antibody can be confirmed by the fact that the reactivity of the antibody to the immobilized methylated protein is inhibited by monomethyllysine, dimethyllysine or trimethyllysine added from the outside, but not inhibited by lysine. The recognition of methyllysine residues without significantly depending on surrounding amino acid sequences can be confirmed by examining the strong activity of the antibody in binding to any proteins obtained by immobilizing different chemically methylated proteins. Alternatively, this can be revealed by the fact that a plurality of proteins can be stained by electrophoresing an animal cell lysate, then transferring it onto a PVDF membrane or nitrocellulose membrane, and staining it by Western blotting with the antibody. Alternatively, this can be revealed by the fact that methylated proteins (e. g. , EF-1α) other than the protein used in immunization are contained in an immuno-precipitate formed upon immuno-precipitation of an animal cell lysate with the anti-methyllysine antibody, or that bands stained with the anti-methyllysine antibody can be detected in an immuno-precipitate with an antibody against EF-1α known to be methylated.

The antibody provided by the present invention is useful in searching for a methyllysine-containing protein as a new target molecule useful in diagnosis and therapy of disease, and upon finding a promising target molecule, can be used for the purpose of regulating its functions or for diagnostic purposes.

### Example 1

### Preparation of anti-methyllysine mouse monoclonal antibody-producing hybridomas

Anti-methyllysine mouse monoclonal antibody-producing hybridomas exemplified by MEK3D7, MEK4E10, MEK5F7, MEK2-5A11 and MEK2-5B11 were prepared by the following method.

These hybridomas have been deposited with International Patent Organisms Depository, National Institute of Advanced Industrial Science and Technology, Japan under Accession Nos. FERM P-19595, FERM P-19596, FERM P-19597, FERM P-19593 and FERM P-19594 respectively since November 21, 2003, then applied for transfer to international deposition and has been deposited under FERM ABP-10168, FERMABP-10169, FERMABP-10170, FERMABP-10166 and FERM ABP-10167 respectively since December 1, 2004.

Mouse immunization was carried out in the following manner. A methylated KLH solution and TiterMax Gold (CytRx Ltd.) were mixed at a ratio of 1 : 1 and then formed into an emulsion by passing the mixture repeatedly through an interchange joint for connecting two injection tubes with each other. The prepared antigen-containing emulsion was used to immunize mice subcutaneously or intraperitoneally 3 or 4 times with 1- to 4-week intervals. The methylated KLH was used as antigen in an amount of 50 to 100 µg per immunization. Bovine serumalbumin (abbreviated hereinafter as BSA) which had been methylated in the same manner as for KLH was immobilized onto an ELISA plate, and when an increase in the antibody titer in blood was confirmed by the following method, the methylated KLH dissolved in PBS was administered intraperitoneally and into a tail vein respectively 4 and 3 days before fusion with myeloma (amount of the methylated KLH administered was 100 µg/mouse).

Measurement of the antibody titer was carried out in the following manner. Bovine serum albumin methylated in the same method as for KLH was dissolved at a concentration of 10 µg/ml in PBS, then added to each well of a 96-well ELISA plate in a volume of 50 µl/well, and adsorbed onto each well overnight at 4°C. Each well was washed with PBS containing 0.05% Tween 20 (PBST) and then used in assay. Blood was collected through a tail vein, then diluted 30-fold with PBST and then separated by centrifugation. The resulting supernatant was used to prepare serial dilutions with PBST, and each dilution was added to each well of the methylated BSA-coated ELISA plate in a volume of 50µl/well. After incubation at room temperature for 30 minutes, each well was washed with PBST, and a horseradish peroxidase (HRP)-labeled anti-mouse IgG solution diluted suitably with PBST was added in a volume of 50 µl/well. After further incubation at room temperature for 30 minutes, o-phenylenediamine was used as substrate and the absorbance at 492 nm was measured to evaluate the activity.

Fusion of the antibody-producing cell with myeloma was carried out in the following manner. Three days after intravenous administration of the antigen, the spleen was removed and spleen cells were isolated. The 5×10⁷ cells were fused with 1×10⁷ cells of separately cultured mouse myeloma SP2/0-Ag14 by using polyethylene glycol. The successfully fused cells were cultured and selected in HAT (hypoxanthine/aminopterin/thymidine) medium. While the cells were cultured for 11 days, half of the medium was exchanged with a fresh medium every several days, and then the antibody titer of the culture supernatant was measured. The cells in the positive wells were cloned by the limiting dilution method to give the objective antibody-producing hybridomas.

The isotypes of the respective antibodies derived from the resulting hybridoma clones MEK3D2, MEK3D7, MEK4E10, MEK5F5, MEK5F7, MEK2-1A3, MEK2-1H9, MEK2-5A11, MEK2-5B11 and MEK2-5G1 were IgG2a κ, IgGl κ, IgGl κ, IgG2a λ, IgG2a λ, IgG2a λ, IgG3 κ, IgG2b κ, IgG2a λ and IgG2a λ, respectively.

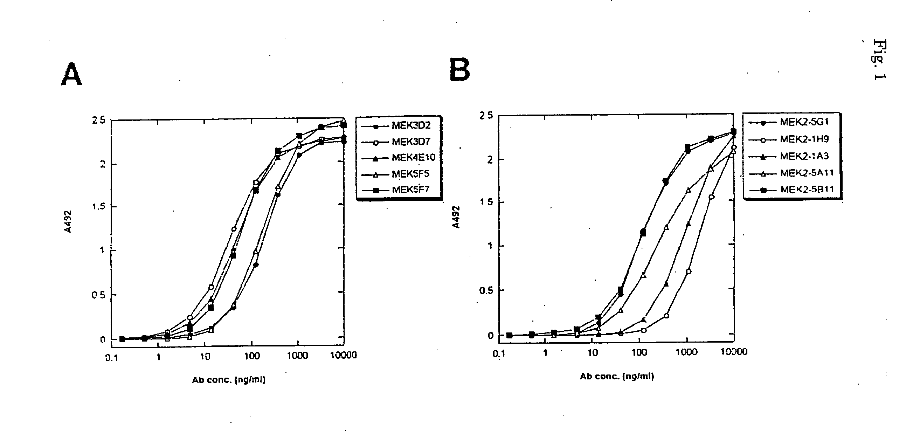

The results of the reactivity, examined by ELISA, of antibodies purified by protein G column from culture supernatants of the hybridomas obtained in this manner are shown in Fig. 1. The method is the same as the method of measuring the antibody titer in blood. As can be seen from the figure, antibodies reacting with the immobilized methylated BSA were contained in any of the culture supernatants.

### Example 2

### Preparation of anti-methyllysine rabbit polyclonal antibodies

The anti-methyllysine rabbit polyclonal antibodies were prepared in the following procedure. Two rabbits (Japanese female white species) were immunized with the methylated KLH in a dose of 0.15mg/rabbit in first immunization and in a dose of 0.3mg/rabbit in second to fifth immunization. The back of the rabbit was administered subcutaneously with the methylated KLH as an emulsion with Freund complete adjuvant in the first immunization and as an emulsion with Freund incomplete adjuvant in the second and subsequent immunization. The immunization was conducted at 2-week intervals, and 1 week after the final immunization, whole blood was collected to prepare antiserum. 118 ml antiserum was obtained in total from the two rabbits.

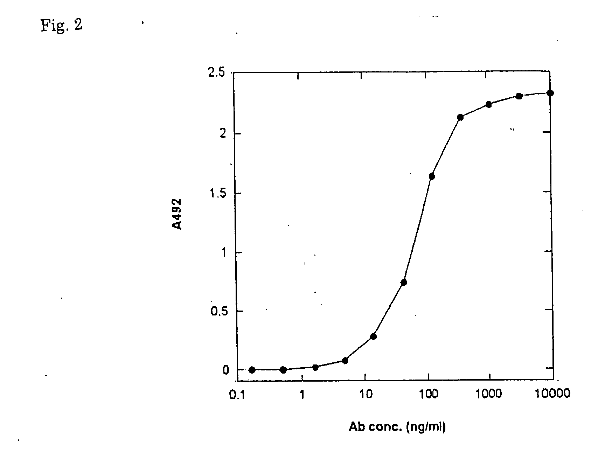

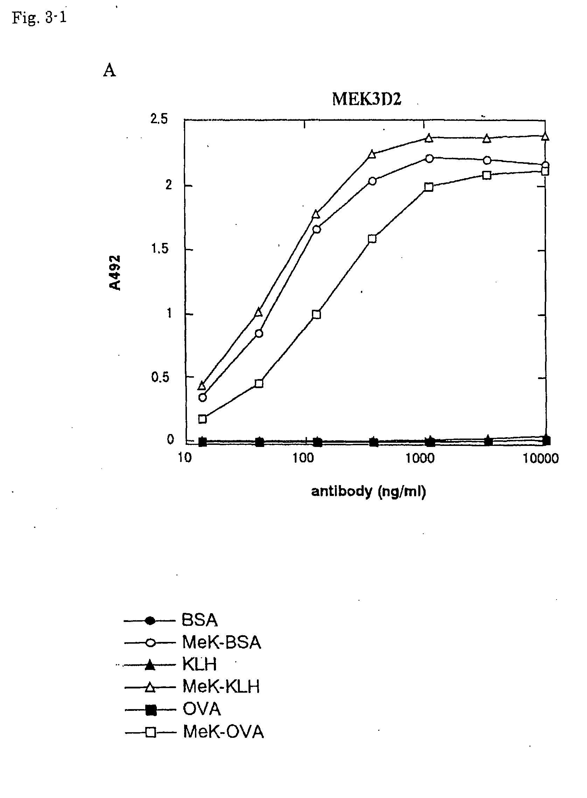

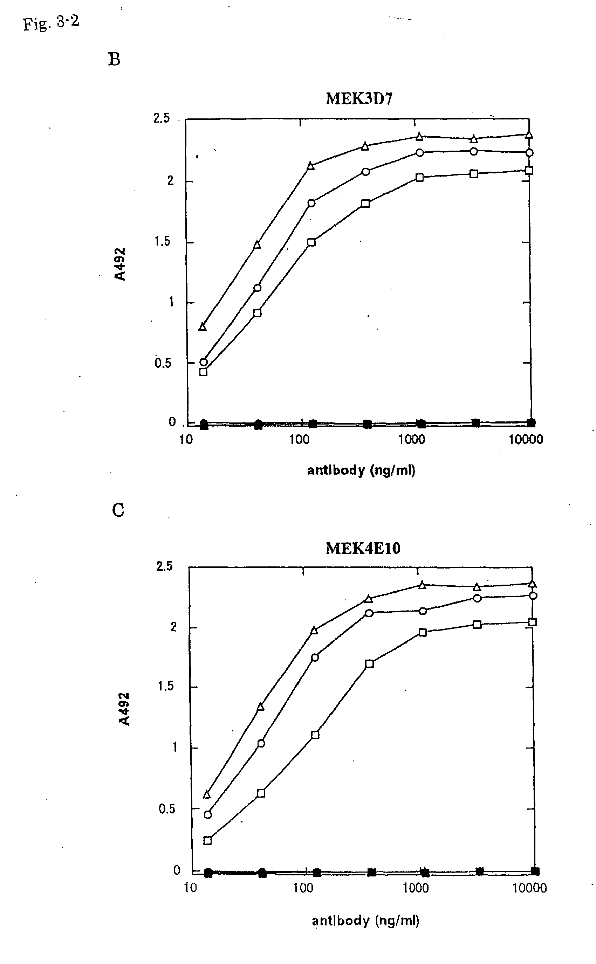

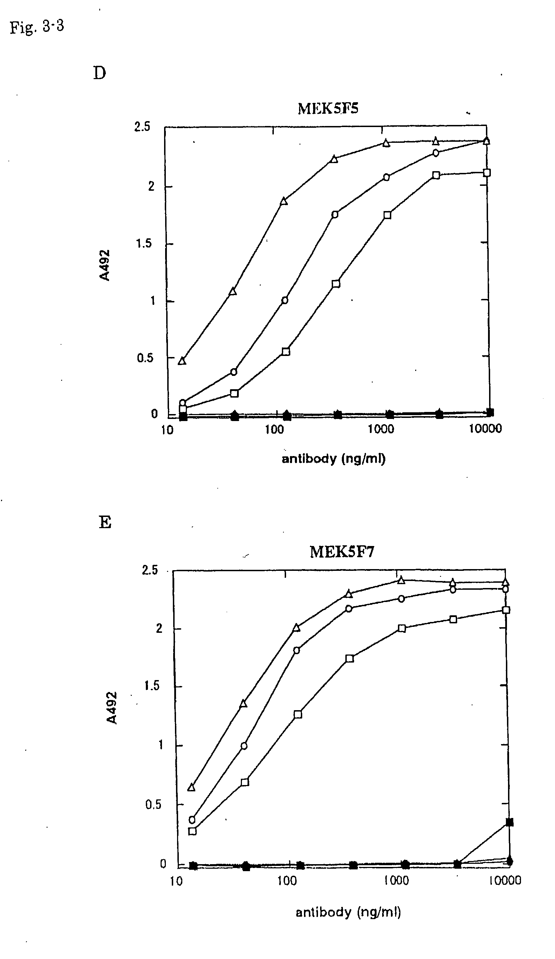

Fig. 2 shows the results of the reactivity, determined by ELISA, of the purified anti-methyllysine rabbit polyclonal antibodies to the immobilized methylated BSA. The antibodies certainly having reactivity could be obtained according to this method.

### Example 3

### Purification of the anti-methyllysine antibodies by an affinity column

The polyclonal antibody-containing antiserum was purified by an affinity column having a methylated protein immobilized thereon.

The affinity column having a methylated protein immobilized thereon was prepared by the following method. First, a carrier having BSA immobilized thereon was prepared in a first step. For immobilization, an Aminolink Immobilization kit available from Pierce was used. 2 ml agarose carrier activated with aldehyde was equilibrated with 5 ml coupling buffer, and then 10 mg BSA dissolved in 2 ml coupling buffer was added. 200 µl reductant solution was added and the BSA was bound to the carrier at room temperature for 6 hours. The carrier was washed with 5 ml coupling buffer, then 5 ml of 1 M ethanolamine was added thereto, and after 200 µl reductant solution was added, the mixture was reacted at room temperature for 30 minutes . After the reaction was finished, the carrier was washed 4 times with 5 ml wash solution and then equilibrated with 0.5 M borate buffer (pH 9.4) . After draining, 2 mg NaBH₄ dissolved in 2 ml borate buffer was added, and 2 µl formalin was added 5 times at 5-minute intervals. The reaction was continued at room temperature for 30 minutes and then the carrier was washed with PBS.

After a column charged with the resultant carrier was equilibrated with PBS, a dilution of 48 ml antiserum with 48 ml PBS was applied onto the column. The column was washed with 10 ml PBS and then subjected to elution with an IgG elution buffer available from Pierce (1 ml×10 fractions). Each fraction was neutralized immediately with 100 µl of 1 M Tris-HCl, pH 7.4. Fractions into which the protein had been eluted were measured for absorbance at 280 nm and then pooled, and the solvent therein was exchanged with PBS by a gel filtration column (PD-10, Amersham Bioscience). 10.2 mg anti-methyllysine rabbit polyclonal antibodies were obtained.

The above affinity column can also be used in purification of monoclonal antibody. One example of the anti-methyllysine mouse monoclonal antibody is shown below. Female Balb/c mice, 7 days after intraperitoneal administration of 0.5 ml pristane to each mouse, was administered intraperitoneally with MEK3D7 hybridoma cells (as 1 ml PBS suspension) in an amount of about 10⁷ cells/mouse. After 9 days, each mouse was sacrificed under anesthesia with ether, and then the ascitic fluid was recovered. 2 ml of the ascitic fluid was diluted twice with PBS and then applied onto the affinity column equilibrated with PBS. After the column was washed with PBS, the protein was eluted with an IgG elution buffer (1 ml/fraction; each fraction was neutralized with 50 µl of 1 M Tris-HCl, pH 8.0). Fractions containing the protein were examined by measuring absorbance at 280 nm and then pooled, and the solvent therein was exchanged with PBS through a gel filtration column (PD-10). 8.5 mg MEK3D7 anti-methyllysine mouse monoclonal antibody was thereby obtained.

### Example 4

### Measurement of the reactivity of the antibodies to various methylated proteins

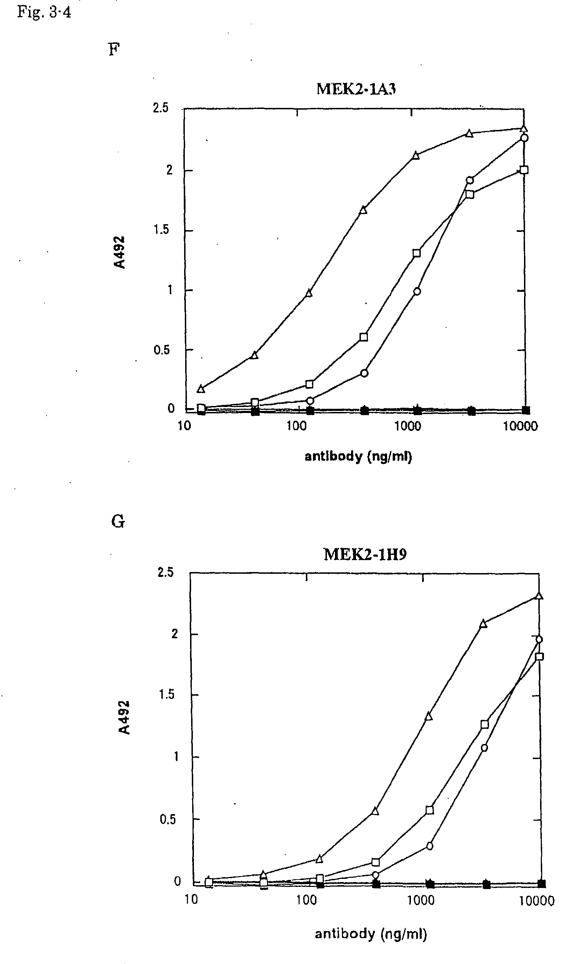

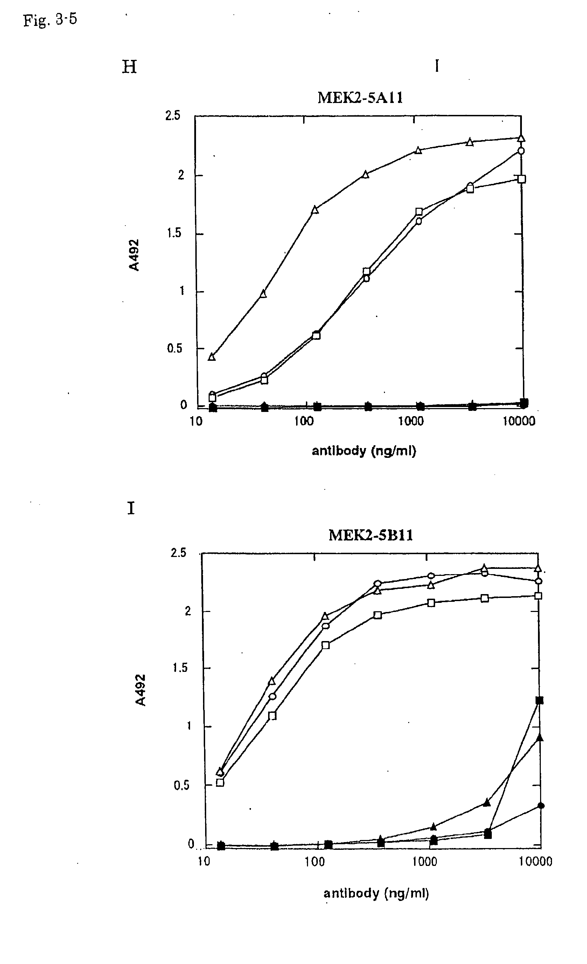

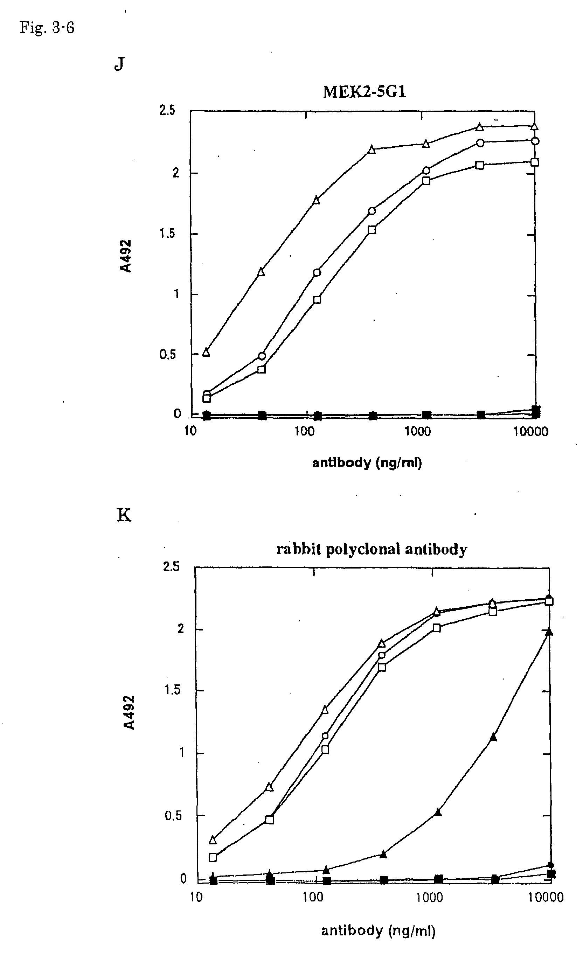

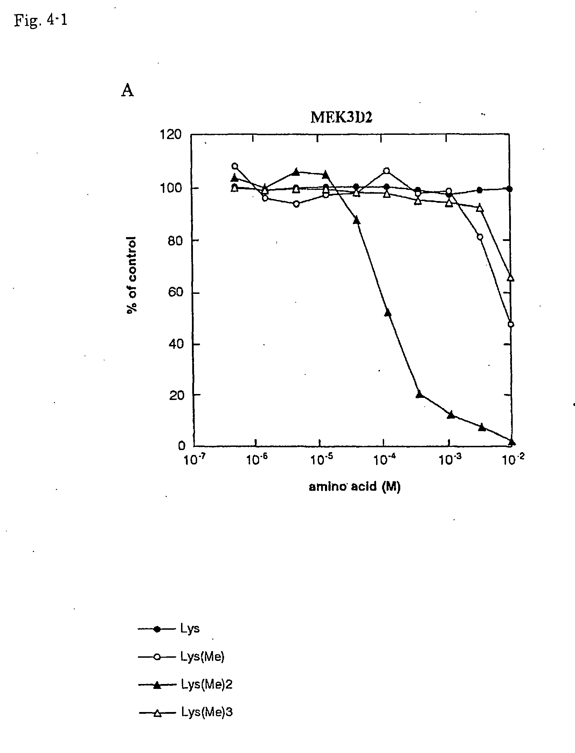

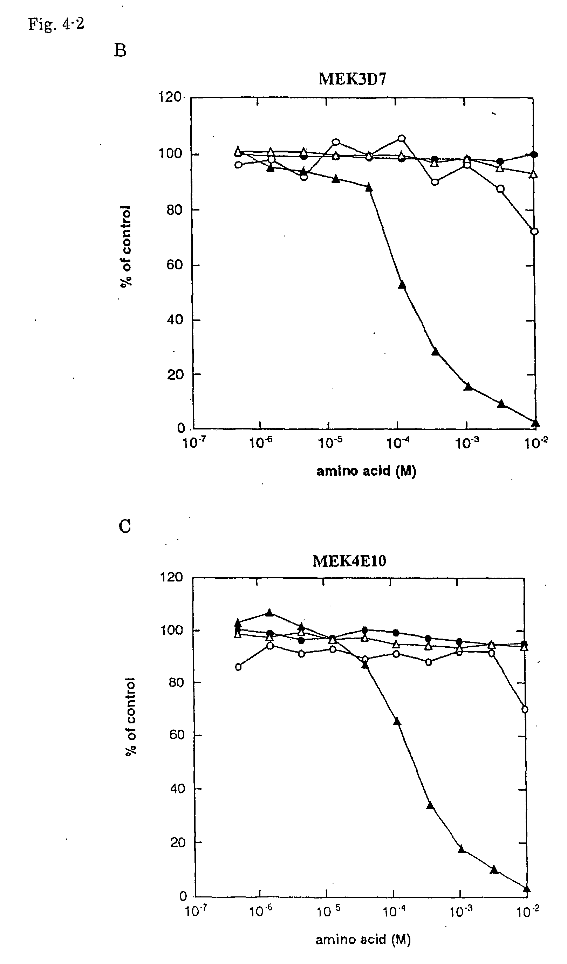

If the resulting antibody can recognize methyllysine without depending on amino acid sequences surrounding methyllysine residues, the antibody must exhibit reactivity to any proteins obtained by chemically methylating arbitrary proteins containing lysine residues. In addition, the reactivity of the antibody to non-methylated proteins must be significantly lower than reactivity to the methylated proteins. Fig. 3 shows the results of examining, by ELISA, how each monoclonal antibody or rabbit polyclonal antibody established this time reacts with proteins BSA, KLH and ovalbumin (OVA) and with immobilized, chemically methylated versions thereof (referred to as MeK-BSA, MeK-KLH and MeK-OVA, respectively) obtained by the above-described method. Both the monoclonal antibodies and polyclonal antibodies exhibited strong reactivity to any of MeK-BSA, MeK-KLH and MeK-OVA, but hardly showed reactivity to the non-methylated proteins except for some cases. Among the monoclonal antibodies, MEK5F7 and MEK2-5B11 at high concentrations exhibited weak reactivity to non-methylated OVA and slightly strong reactivity to the 3 non-methylated proteins, respectively, but the concentration of the antibodies exhibiting such reactivity should be at least 1000 times as high as the concentration of the antibodies exhibiting equivalent reactivity to the methylated proteins, and thus any of these antibodies can be said to be methylated site-specific antibodies. On the other hand, the rabbit polyclonal antibodies showed significantly strong reactivity to non-methylated KLH. The reason for this phenomenon is not evident, but from the viewpoint of the necessary concentration of the antibodies, they can still be said to be methylated site-specific antibodies. If it is desired to remove the antibodies showing such unspecific reaction, they may be absorbed onto a column having non-methylated KLH immobilized thereon.

### Example 5

### Confirmation of the inhibition, by methyllysine, of antibody reaction

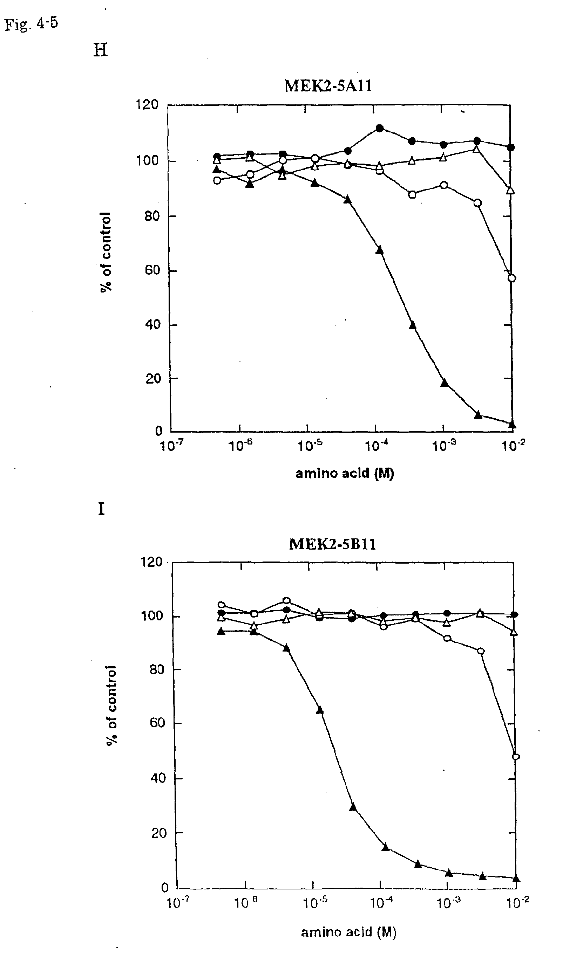

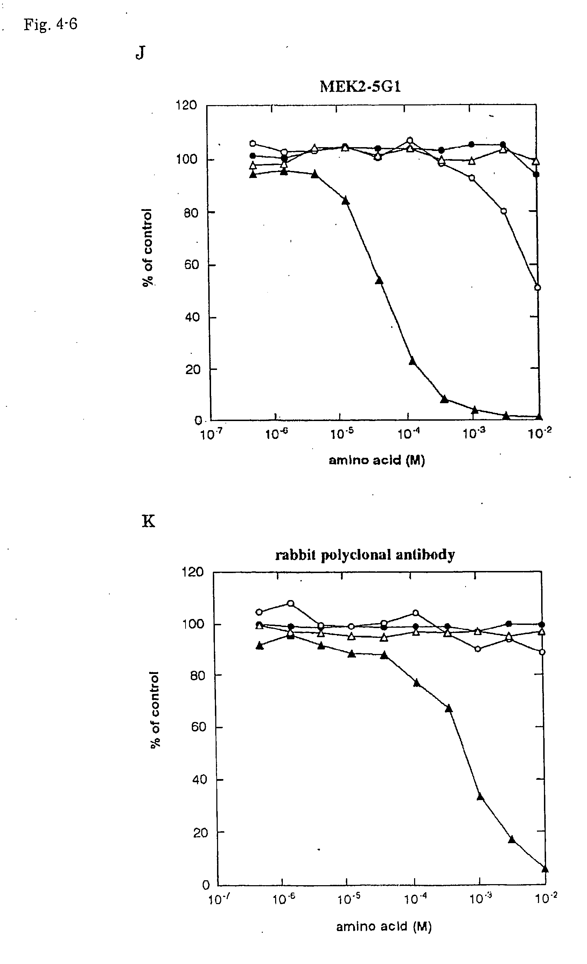

As lysine methylated at s-amino group, there are 3 kinds of lysines, that is, monomethyllysine, dimethyllysine and trimethyllysine. A competitive experiment was carried out using each of the modified amino acids in order to confirm which of these lysines the antibody has stronger reactivity to or how specific for methyllysine the antibody is. 10 µg/ml methylated BSA was used in a volume of 50 µl/well to adsorb the antigen onto an ELISA plate, and then 1 µg/ml of each antibody was reacted in the presence of modified or unmodified lysine at varying concentrations, and the amount of the antibody which could bind to the solid phase was detected with HRP-labeled anti-mouse IgG antibody (substrate: o-phenylenediamine). The results are shown in Fig. 5. Both the monoclonal antibodies and polyclonal antibodies underwent highest competition with dimethyllysine. Some monoclonal antibodies underwent further competition with monomethyllysine, and MEK3D2 was observed to undergo further weak competition with trimethyllysine. However, none of the antibodies underwent competition with unmodified lysine. In the concentration range examined, the polyclonal antibodies underwent competition with dimethyllysine only.

The above results indicate that any antibodies established this time recognize methyllysine and tend to show particularly high specificity for dimethyllysine.

### Example 6

### Analysis of an animal cell lysate by Western blotting

The analysis of an animal cell lysate by Western blotting was carried out in order to confirm whether or not the established antibodies could detect various methyllysine-containing proteins estimated to occur in animal cells. Human T cell leukemic cell line MOLT-4F was lysed with 2% SDS in 62.5 mM Tris-HCl, pH 6.8, then applied in an amount of 30 µg/lane onto 12% SDS-polyacrylamide gel and electrophoresed. After proteins separated by electrophoresis were transferred onto a PVDF membrane, each antibody dissolved at a concentration of 1 µg/ml in TBST was incubated with the membrane having the proteinstransferred thereon. After 30 minutes, the membrane was washed, and the mouse monoclonal antibodies were reacted with HRP-labeled anti-mouse IgG, while the rabbit polyclonal antibodies were reacted with HRP-labeled anti-rabbit IgG, and each membrane was washed, and then bands thereon with ECL Plus chemoluminescence reagent from Amersham Bioscience were detected. The results are shown in Fig. 5. As shown in the figure, any mouse monoclonal antibodies shown therein have detected bands considered as a plurality of methylated proteins and revealed to be useful in detecting intracellular methylated proteins. Although not shown in data, it was confirmed that other anti-methyllysine monoclonal antibodies can also similarly detect bands considered as a plurality of methylated proteins. For comparison, the results of the reaction, at the same concentration, of an anti-methyllysine rabbit polyclonal antibody commercially available from Abcam Ltd. are also shown, but detectable bands are not so many, as compared with the monoclonal antibodies or polyclonal antibodies established this time, and it came to be revealed that by using the method shown this time, more excellent antibodies can be obtained. The band between the molecular weights of 14.4 k and 21.5 k is a band of histone, and from comparison of the intensity of this band, it appears that the anti-methyllysine antibodies established this time, regardless of whether they are monoclonal or polyclonal antibodies, are superior to the commercial antibodies.

### Example 7

### Analysis of an animal cell lysate by immune precipitation

To confirm whether the methylation of a molecule previously known to be methylated can be detected by the established antibodies, a confirmation experiment was carried out using immune precipitation.

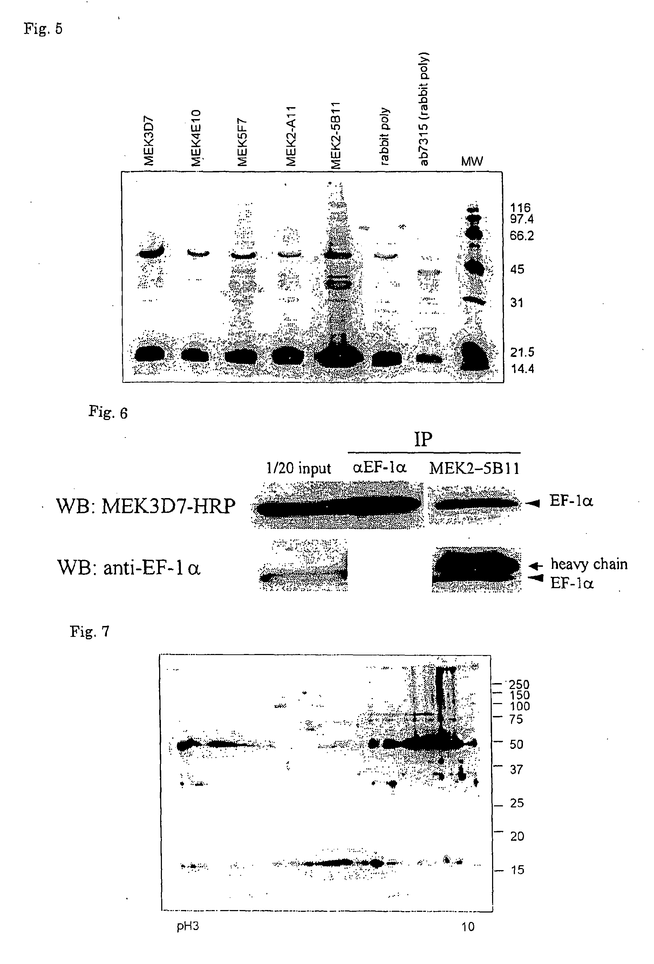

Elongation factor 1α (EF-1α) is a protein having a molecular weight of about 50 kD, known to be methylated at several sites in the molecule thereof, and this protein corresponds highly probably to a band at a position of a molecular weight in the vicinity of 50 kD in Fig. 5. To confirm this, MOLT-4F cells were lysed with a lysis buffer (120 mM NaCl, 1 mM CaCl₂, 0.5% Nonidet P-40, 50 mM Tris-HCl, pH 8.0) containing a protease inhibitor mixture (Complete Mini EDTA-free, Roche) and then immune-precipitatedwith the anti-methyllysine antibody MEK2-5B11 or a commercial anti-EF-1α antibody (clone CBP-KK1, Upstate Ltd.). The immune precipitate was developed with SDS polyacrylamide electrophoresis, then transferred onto a PVDF membrane and detected with HRP-labeled anti-methyllysine antibody MEK3D7 or anti-EF-1α antibody. When the anti-EF-1α antibody was used, HRP-labeled anti-mouse IgG was used as secondary antibody. Coloration was carried out with ECL plus available from Amersham Bioscience. The results are shown in Fig. 6. This figure is an enlargement in the vicinity of molecular weight 50 kD. As shown in the figure, the immune precipitate with the anti-methyllysine antibody MEK2-5B11 was detected with another anti-methyllysine antibody MEK3D7 and also with the antibody against EF-1α. On one hand, the immune precipitate with the anti-EF-1α antibody was detected with the anti-methyllysine antibody MEK3D7. These results reveal that both the anti-methyllysine antibodies MEK3D7 and MEK2-5B11 established this time can recognize a methylated site of EF-1α. This result suggests that the band with a molecular weight of about 50 kD in Fig. 5 is a band of EF-1α, and by comparing the ability to detect this band, both the monoclonal antibodies and polyclonal antibodies established this time were found to be superior to the commercial anti-methyllysine rabbit polyclonal antibody.

### Example 8

### Analysis of intracellular methyllysine-containing proteins by two-dimensional electrophoresis

MOLT-4F cells were lysed with 9.8 M urea, 0.5% CHAPS, 10 mM DTT, and then 50 µg proteins were subjected to isoelectric focusing in one-dimensional development in the range of pH 3 to 10 and further separated by the electrophoresis on 12% SDS polyacrylamide gel. After transfer to a PVDF membrane, the proteins were detected with HRP-labeled anti-methyllysine antibody MEK3D7. The results are shown in Fig. 7. As can be seen from the figure, spots of a plurality of methyllysine-containing proteins can be detected with this antibody.

### Brief Description of Drawings

Fig. 1 shows the results of examination, by ELISA, of the reactivity of anti-methyllysine mouse monoclonal antibodies to immobilized methylated BSA. (A) is the result of mouse monoclonal antibodies produced by clones MEK3D2, MEK3D7, MEK4E10, MEK5F5, and MEK5F7, and (B) is the result of mouse monoclonal antibodies produced by clones MEK2-5G1, MEK2-1H9, MEK2-1A3, MEK2-5A11, and MEK2-5B11.
Fig. 2 shows the results of examination, by ELISA, of the reactivity of anti-methyllysine rabbit polyclonal antibodies to immobilized methylated BSA.
Fig. 3-1 shows the results of examination, by ELISA, of the reactivity of the anti-methyllysine monoclonal antibody MEK3D2 to methylated or non-methylated BSA, KLH and OVA.
Fig. 3-2 shows the results of examination, by ELISA, of the reactivity of the anti-methyllysine monoclonal antibodies (B) MEK 3D7 and (C) MEK 4E10 to methylated or non-methylated BSA, KLH and OVA.
Fig. 3-3 shows the results of examination, by ELISA, of the reactivity of the anti-methyllysine monoclonal antibodies (D) MEK 5F5 and (E) MEK 5F7 to methylated or non-methylated BSA, KLH and OVA.
Fig. 3-4 shows the results of examination, by ELISA, of the reactivity of the anti-methyllysine monoclonal antibodies (F) MEK 2-1A3 and (G) MEK 2-1H9 to methylated or non-methylated BSA, KLH and OVA.
Fig. 3-5 shows the results of examination, by ELISA, of the reactivity of the anti-methyllysine monoclonal antibodies (H) MEK 2-5A11 and (I) MEK 2-5B11 to methylated or non-methylated BSA, KLH and OVA.
Fig. 3-6 shows the results of examination, by ELISA, of the reactivity of the anti-methyllysine monoclonal antibodies (J) MEK 2-5G1 and (K) rabbit polyclonal antibody to methylated or non-methylated BSA, KLH and OVA.

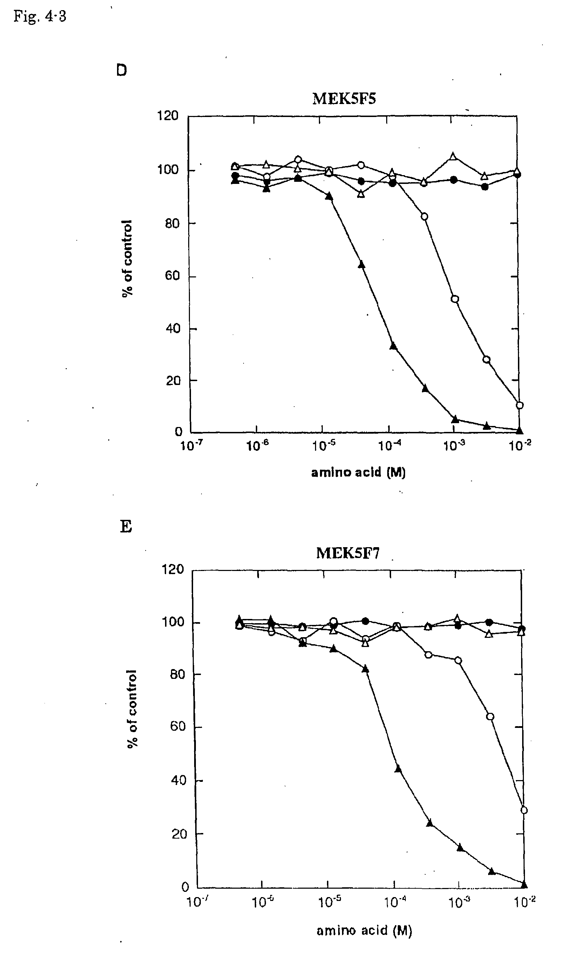

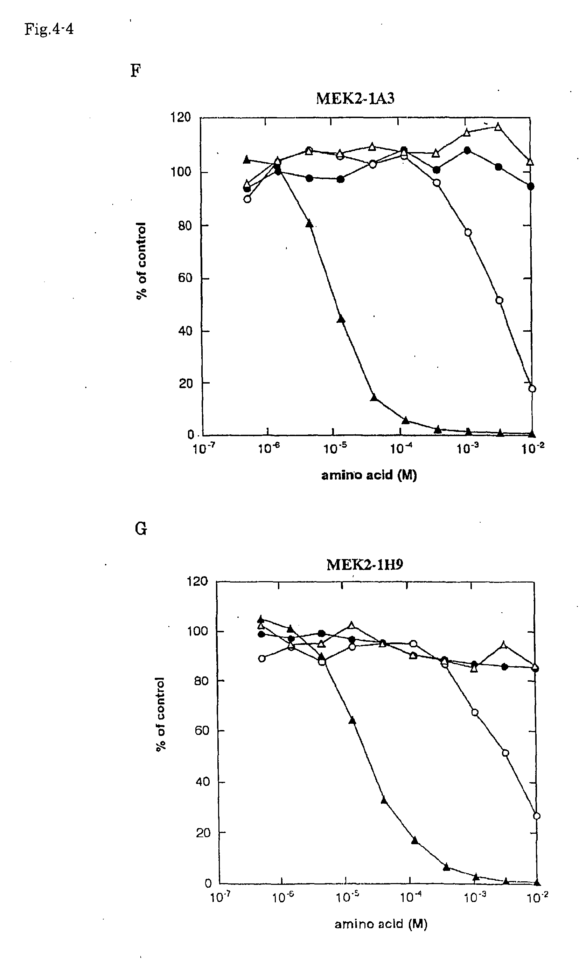

Fig. 4-1 shows the results of inhibition, by lysine, monomethyllysine, dimethyllysine and trimethyllysine, of the reactivity of the anti-methyllysine antibody MEK3D2 to immobilized methylated BSA.
Fig. 4-2 shows the results of inhibition, by lysine, monomethyllysine, dimethyllysine and trimethyllysine, of the reactivity of the anti-methyllysine antibodies (B) MEK 3D7 and (C) MEK 4E10 to immobilized methylated BSA.
Fig. 4-3 shows the results of inhibition, by lysine, monomethyllysine, dimethyllysine and trimethyllysine, of the reactivity of the anti-methyllysine antibodies (D) MEK 5F5 and (E) MEK 5F7 to immobilized methylated BSA.
Fig. 4-4 shows the results of inhibition, by lysine, monomethyllysine, dimethyllysine and trimethyllysine, of the reactivity of the anti-methyllysine antibodies (F) MEK 2-1A3 and (G) MEK 2-1H9 to immobilized methylated BSA.
Fig. 4-5 shows the results of inhibition, by lysine, monomethyllysine, dimethyllysine and trimethyllysine, of the reactivity of the anti-methyllysine antibodies (H) MEK 2-5A11 and (I) MEK 2-5B11 to immobilized methylated BSA.
Fig. 4-6 shows the results of inhibition, by lysine, monomethyllysine, dimethyllysine and trimethyllysine, of the reactivity of the anti-methyllysine antibodies (J) MEK 2-5G1 and (K) rabbit polyclonal antibody to immobilized methylated BSA.
Fig. 5 shows the results of detection of methyllysine-containing proteins in an MOLT-4F cell lysate by Western blotting with each monoclonal antibody and polyclonal antibody.
Fig. 6 shows the results of detection, by Western blotting with an anti-methyllysine antibody and anti-EF-1α antibody, of immune-precipitates of an MOLT-4F cell lysate with an anti-methyllysine antibody or anti-EF-1α antibody.
Fig. 7 shows the results of detection with HRP-labeledMEK3D7 antibody after development of an MOLT-4F cell lysate by two-dimensional electrophoresis.

## Claims

1. An anti-methyllysine antibody capable of specifically recognizing methyllysine and not recognizing lysine.

2. An anti-methyllysine antibody capable of specifically recognizing a methyllysine residue in a protein, without being influenced by surrounding amino acid residues.

3. An anti-methyllysine antibody specifically binding to dimethyllysine and monomethyllysine.

4. The antibody according to any of claims 1 to 3, whose reactivity to dimethyllysine is superior to reactivity to monomethyllysine.

5. The antibody according to any of claims 1 to 4, which is a polyclonal antibody.

6. The antibody according to any of claims 1 to 4, which is a monoclonal antibody.

7. A hybridoma producing an anti-methyllysine antibody and selected from the group consisting of MEK3D7, MEK4E10, MEK5F7, MEK2-5A11 and MEK2-5B11.

8. An anti-methyllysine mouse monoclonal antibody produced by the hybridoma of claim 7.

9. A process for producing the polyclonal antibody of claim 5, which comprises immunizing an animal with an antigen obtained by chemically methylating a protein and subjecting the resulting antibody to affinity purification with methyllysine or a protein obtained by chemically methylating a protein different from the antigen.

10. A process for producing the monoclonal antibody of claim 6, which comprises immunizing an animal with an antigen obtained by chemically methylating a protein and then selecting a hybridoma secreting an antibody recognizing a protein obtained by chemically methylating a protein different from the antigen.

11. A method of detecting a methylated protein, which comprises using the antibody of any of claims 1 to 6 or 8.
